Europäisches Patentamt

European Patent Office

Office européen des brevets

(11) Publication number: 0 657 160 A1

# (12) EUROPEAN PATENT APPLICATION

(21) Application number: 94203476.0

(22) Date of filing: 30.11.94

(51) Int. Cl.⁶: **A61K 7/24, A61K 7/16**

(30) Priority: **09.12.93 EP 93309903**

(43) Date of publication of application:
**14.06.95 Bulletin 95/24**

(84) Designated Contracting States:
**AT BE CH DE DK ES FR GB GR IE IT LI NL PT SE**

(71) Applicant: **UNILEVER N.V.**
**P.O. Box 760**
**NL-3000 DK Rotterdam (NL)**

(84) **BE CH DE DK ES FR GR IT LI NL PT SE AT**

(71) Applicant: **UNILEVER PLC**
**Unilever House**
**Blackfriars**
**P.O. Box 68**
**London EC4P 4BO (GB)**

(84) **GB IE**

(72) Inventor: **Stead, Willem John, Unilever Res**
**Port Sunlight Lab**
**Ouarry Road,**
**East**
**Bebington Wirral Merseyside L63 3JW (GB)**
Inventor: **Klugkist, Jan, Unilever Res**
**Vlaardingen Lab**
**Olivier van Noortlaan 120**
**NL-3133 AT Vlaardingen (NL)**
Inventor: **Peacock, Jillian Mary**
**32 Alwyn Drive**
**East Kilbride (GB)**
Inventor: **Orchardson, Robin**
**University of Glasgow Institute of Physiology**
**Glasgow G12 8OO (GB)**

(74) Representative: **van Gent, Jan Paulus**
**Unilever N.V.,**
**Patent Division**
**Postbus 137**
**NL-3130 AC Vlaardingen (NL)**

(54) Oral composition for desensitising sensitive teeth.

(57) The present invention relates to oral compositions for desensitising sensitive teeth. By using potassium tartrate as desensitising agent in the manufacture of oral compositions for desensitising sensitive teeth, a composition with improved desensitising properties is obtained. In a preferred embodiment the compositions also contain a zinc salt.

This invention relates to an oral composition for desensitising sensitive teeth. More particularly, it relates to the use of potassium tartrate as desensitising agent in the manufacture of an oral desensitising composition.

Tooth hypersensitivity is a problem that affects many people. People suffering from tooth hypersensitivity experience a painful reaction when the teeth are exposed e.g. to hot, cold, sweet, acid, pressure and the like stimuli. They may experience this painful reaction particularly when the normal protective enamel sheathing of the teeth is damaged or when the gums have receded, exposing tooth dentine.

The present invention has as its object to mitigate the problem of sensitive teeth by using an effective amount of potassium tartrate as desensitising agent in the manufacture of an oral desensitising composition.

According to the present invention, potassium tartrate is used as desensitising agent in the manufacture of an oral composition for use against sensitive teeth.

Several potassium salts such as potassium nitrate, potassium citrate, potassium oxalate, potassium chloride or potassium bicarbonate have been proposed as desensitising agents in oral compositions. US-A-3 863 006 discloses the use of potassium nitrate as a tooth desensitising agent. Potassium citrate has been disclosed in European Patent Application No. 0 095 871 to be effective to reduce the pain associated with sensitive teeth. Potassium oxalate has been disclosed in US-A-4 057 621. The use of potassium chloride and potassium bicarbonate has been similarly disclosed in International Patent Application WO 85/04098.

In US-A-4 656 031, Example 5, Table VI a toothpaste with 1.01% potassium tartrate is described. The purpose of the use of this ingredient is, however, entirely different from that according to the present invention; it is used according to this reference to adjust the lamellar spacing of the liquid lamellar crystal surfactant phase in toothpastes.

Finally, Maita et al in a Poster, POS 20, presented for an IADR/AADR Satellite Symposium on 14-16 March 1993 in Georgia, USA, have reported on the effects of i.a. a 30% potassium tartrate solution on the permeation of magnesium ions through radicular dentine in extracted bovine teeth, and found that this potassium tartrate solution did not significantly reduce the permeation of magnesium ions radicular dentine.

The potassium tartrate is used according to the present invention in an effective amount. This amount ranges from 0.2 to 10% by weight, preferably from 0.5-7.5% by weight and particularly preferably from 1.0-6.5% by weight of the oral composition. The potassium tartrate may be mono- or dipotassium tartrate, dipotassium tartrate being preferred.

The oral compositions can be formulated in any suitable application form, such as gels, mouthwashes and toothpastes. The oral care compositions of the present invention may furthermore comprise optional, conventional ingredients such as pharmaceutically acceptable carriers like starch, sucrose, water or water/alcohol systems etc.. Small amounts of surfactants may also be included, such as anionic, nonionic and amphoteric surfactants. When formulated into a dentifrice, such formulation may contain all the usual dentifrice ingredients. Thus, they may comprise particulate abrasive materials such as silicas, aluminas, calcium carbonates, dicalciumphosphates, calcium pyrophosphates hydroxyapatites, trimetaphosphates, insoluble hexametaphosphates and so on, usually in amounts between 5 and 60% by weight.

Furthermore, the dentifrice formulations may comprise humectants such as glycerol, sorbitol, propyleneglycol, xylitol, lactitol and so on.

Binders and thickeners such as sodium carboxymethyl-cellulose, xanthan gum, gum arabic etc. may also be included, as well as synthetic polymers such as polyacrylates and carboxyvinyl polymers such as Carbopol®.

Flavours such as peppermint and spearmint oils may also be included, as well as preservatives, opacifying agents, colouring agents, pH-adjusting agents, sweetening agents and so on.

Anti-bacterial agents may also be included such as Triclosan, chlorhexidine, copper-, zinc- and stannous salts such as zinc citrate, sodium zinc citrate and stannous pyrophosphate, sanguinarine extract, metronidazole. Further examples of anti-bacterial agents are quaternary ammonium compounds such as cetylpyridinium chloride; bis-guanides such as chlorhexidine digluconate, hexetidine, octenidine, alexidine; halogenated bisphenolic compounds such as 2,2' methylenebis-(4-chloro-6-bromophenol). In this respect it is a preferred embodiment of the present invention that potassium tartrate can be beneficially included in an oral composition which also contains a zinc salt such as zinc citrate to provide a composition which has a desensitising effect and also an anti-gingivitis effect, the potassium tartrate not interfering with the antibacterial efficacy of the zinc salt.

Polymeric compounds which can enhance the delivery of active ingredients such as anti-bacterial agents can also be included. Examples of such polymers are copolymers of polyvinylmethylether with maleic anhydride and other similar delivery enhancing polymers, e.g. those described in DE-A-3,942,643 (Colgate)

Furthermore anti-inflammatory agents such as ibuprofen, flurbiprofen, aspirin, indomethacin etc. may also be included.

Anti-caries agents such as sodium- and stannous fluoride, aminefluorides, monosodiumfluorophosphate, casein, plaque buffers such as urea, calcium lactate, calcium glycerophosphate, strontium polyacrylates may also be included. Other optional ingredients include vitamins such as Vitamin C, and plant extracts. Additional desensitising agents such as potassium citrate, potassium chloride, potassium bicarbonate, potassium oxalate, potassium nitrate as well as strontium salts may also be included.

Buffers and salts to buffer the pH and ionic strength of the compositions may also be included. Liposomes and other encapsulates may also be used to improve delivery or stability of active ingredients.

Furthermore, the oral compositions may comprise anti-calculus agents such as alkalimetal pyrophosphates, hypophosphite-containing polymers, organic phosphonates, phosphocitrates etc..

In addition, the compositions may comprise functional biomolecules such as bacteriocins, antibodies, enzymes and so on.

Other optional ingredients that may be included are e.g. bleaching agents such as peroxy compounds e.g. potassium peroxydiphosphate, effervescing systems such as sodium bicarbonate/citric acid systems, colour change systems, and so on.

When formulated as a mouthwash, the oral care composition usually comprises a water/alcohol solution, flavour, humectant, sweetener and colorant.

The present invention will further be illustrated by way of Example.

**Example I**

Using the method as described in J. Physiol <u>275</u>, (1978), pages 177-189, experiments were carried out in vitro on spinal nerve roots of the cauda equina dissected from killed rats. Compound nerve action potentials (CAPs) were recorded from the nerves which were placed in a bath incorporating stimulating and recording electrodes and analyzed with MacLab Scope. Kreb's solutions containing various potassium ion concentrations were applied for 5 minutes to an isolated central portion of the nerve between the two sets of electrodes. With all potassium salts tested, the amplitude and area of the CAPs were reduced in a dose-dependent manner by test solutions containing 8 mM $K^+$ or more. The results are shown in the table below. "Mean INT" in the table is the mean integrated CAP, expressed as a percentage of the control response, i.e. the response obtained with Krebs' solution containing 4mM $K^+$. There were no differences in the results for CAP peak amplitudes and integrated CAPs.

| K-SALT | [K+] mM | mean INT.(%) | S.D. |
|---|---|---|---|
| $K_2$ Tartrate (n = 9) | 8 | 83 | 19 |
| | 16 | 15 | 24 |
| | 24 | 2 | 2 |
| | 32 | 1 | 1 |
| | 64 | 1 | 1 |
| $K_2$ Oxalate (n = 8) | 8 | 84 | 9 |
| | 16 | 42 | 27 |
| | 24 | 11 | 13 |
| | 32 | 3 | 4 |
| | 64 | 1 | 1 |
| KCl (n = 18) | 8 | 98 | 5 |
| | 16 | 59 | 24 |
| | 24 | 13 | 20 |
| | 32 | 1 | 2 |
| | 64 | 0 | 0 |
| $KNO_3$ (n = 12) | 8 | 91 | 10 |
| | 16 | 54 | 29 |
| | 24 | 17 | 19 |
| | 32 | 2 | 4 |
| | 64 | 0 | 0 |

Wilcoxons' Sum of Ranks test was used to compare the % of control I-CAP values obtained after 5 min exposure to the different $K^+$ salts at equivalent concentrations. NS represents no significant difference between the CAP values, * shows a significance level of < 0.05, ** is < 0.01; and *** is < 0.002.

|  | $K^+mM$ | KCL | K tart | K ox | KNO$_3$ |
|---|---|---|---|---|---|
| KCl | 8 |  | ** | ** |  |
|  | 16 |  | *** | NS |  |
|  | 24 |  | NS | NS | all NS |
|  | 32 |  | NS | ** |  |
|  | 64 |  | NS | *** |  |
| K tart | 8 |  |  | NS | NS |
|  | 16 |  |  | * | *** |
|  | 24 |  |  | * | NS |
|  | 32 |  |  | * | NS |
|  | 64 |  |  | * | NS |
| K ox | 8 |  |  |  | NS |
|  | 16 |  |  |  | NS |
|  | 24 |  |  |  | NS |
|  | 32 |  |  |  | NS |
|  | 64 |  |  |  | *** |

At concentrations of 16-64 mM $K^+$, the effect of potassium tartrate was significantly greater than that of potassium oxalate ($p < 0.05$). From the above dose-response curves, 50% reduction of the CAP was produced by 11.6 mM $K^+$ when present as potassium tartrate, and by 14.3 mM $K^+$ when present as potassium oxalate. In comparison, 50% CAP reduction occurred with 17.8 mM $K^+$ as KCl and 17.4 mM $K^+$ as KNO$_3$

**Example 2**

The following are formulations useful in the present invention.

| Mouthwash | % by weight |
|---|---|
| dipotassium tartrate monohydrate | 2.18 |
| sodium fluoride | 0.05 |
| sorbitol (70% w/w) | 7.00 |
| ethanol (100% w/w) | 6.00 |
| ethoxylated castor oil | 0.45 |
| flavour | 0.20 |
| sodium saccharine | 0.05 |
| colour | 0.05 |
| demineralized water | 84.02 |

| Toothpaste | % by weight |
|---|---|
| dipotassium tartrate monohydrate | 5.83 |
| sodium monofluorophosphate | 0.80 |
| zinc citrate trihydrate | 0.75 |
| triclosan | 0.30 |
| abrasive silica | 5.00 |
| thickening silica | 8.00 |
| sorbitol (70% syrup) | 45.00 |
| polyethyleneglycol (MW 1500) | 5.00 |
| sodium laurylsulphate | 1.50 |
| flavour | 1.20 |
| saccharin | 0.20 |
| sodium carboxymethylcellulose | 0.80 |
| titanium dioxide | 0.50 |
| water | 25.12 |

**Example 3**

The following mouthwash formulation was tested in pH-stat studies to determine the bio-activity of zinc (% inhibition of acid production) in accordance with the procedure as described by Watson, Cummins and Van der Ouderaa in Caries Research <u>25</u> (1991) pages 431-437, except that 0.05 ml of the mouthwash formulation were tested per 5 ml of incubation mixture.

| | % by weight |
|---|---|
| glycine | 0.50 |
| zinc sulphate. $7H_2O$ | 0.20 |
| sodium fluoride | 0.05 |
| sorbitol (70% syrup) | 7.00 |
| ethanol (100% w/w) | 6.00 |
| flavour | 0.20 |
| colour | 0.0075 |
| ethoxylated castor oil | 0.45 |
| sodium saccharin | 0.04 |
| dipotassium tartrate | 2.18 |
| demineralised water | q.s. |

The bio-activity of the zinc was found to be 65%, which, when compared with the same formulation without the dipotassium tartrate which also had a value of 65%, showed that the zinc bioactivity was fully retained.

**Claims**

1. Use of an effective amount of potassium tartrate as desensitising agent in the manufacture of an oral desensitising composition.

2. Use according to claim 1, characterised in that the potassium tartrate is used in an amount of 0.2-10% by weight, calculated on the oral composition.

3. Use according to claim 1 or 2, characterised in that the oral composition further comprises a zinc salt.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP-A-0 404 558 (YISSUM RESEARCH DEVELOPMENT COMPANY OF THE HEBREW UNIVERSITY OF JERUSA) * example 17; table 17 * | 1,2 | A61K7/24 A61K7/16 |
| A | DATABASE WPI Week 8951, Derwent Publications Ltd., London, GB; AN 89-376247 & RO-A-97 282 * abstract * | 1,2 | |
| A | FR-A-496 422 (RHEIN) * the whole document * | 1,2 | |
| A,D | EP-A-0 095 871 (RECKITT AND COLMAN PRODUCTS) * the whole document * | 1,2 | |
| A | US-A-4 937 066 (VLOCK) * the whole document * | 1-3 | TECHNICAL FIELDS SEARCHED (Int.Cl.6) A61K |

The present search report has been drawn up for all claims

| Place of search | Date of completion of the search | Examiner |
|---|---|---|
| THE HAGUE | 15 March 1995 | Fischer, J.P. |

EPO FORM 1503 03.82 (P04C01)